# EUROPEAN PATENT SPECIFICATION

(11) **EP 1 174 111 B1**
(45) Date of publication and mention of the grant of the patent: **26.01.2005**
(21) Application number: 01116741.8
(22) Date of filing: 19.07.2001
(51) Int. Cl.: A61K 7/06, A61K 7/50

(54) **Hair cosmetic composition**
Kosmetische Zusammensetzung für das Haar
Composition cosmétique pour la chevelure

(30) Priority: 21.07.2000 JP 2000220666
(43) Date of publication of application: 23.01.2002
(73) Proprietor: Kao Corporation, Tokyo 103-8210 (JP)
(72) Inventor: Morita, Kouzi, Kao Corporation Research Lab., Tokyo 131-8501 (JP); Terazaki, Hiroyuki, Kao Corporation Research Lab., Sumida-ku, Tokyo 131-8501 (JP)
(74) Representative: HOFFMANN - EITLE

(56) References cited:
- EP-A- 0 407 040
- EP-A- 0 615 741
- WO-A-01/08654
- US-A- 4 892 728
- US-A- 5 213 792
- US-A- 5 693 317
- US-A- 5 951 993

## Description

### Technical Field

The present invention relates to a hair cosmetic composition which is excellent in smoothness and flexibility during the period from its application to the hair until washing-off (rinsing) and also excellent in softness and moisturized feeling after drying, particularly, excellent in the effect of retaining slick and smooth touch upon application even until rinsing.

### Background Art

For improving the touch of hair after shampooing, hair cosmetic compositions such as rinse, conditioner and treatment have been used. These hair cosmetic compositions contain a cationic surfactant, but since an improvement in the touch such as flexibility is insufficient, a higher alcohol is used in combination.

This higher alcohol imparts the hair with flexibility and oil feel, thereby attaining improvement in the touch. High melting point of it however makes it difficult to prepare a hair cosmetic composition and in addition, a hair cosmetic composition prepared using it has a problem in stability (Japanese Patent Application Laid-Open No. 2000-72628).

Many hair cosmetic compositions contain a silicone derivative to impart the dried hair with grease-free smoothness, but they are accompanied with the problem that owing to the incorporation of the silicone derivative, friction resistance occurs between hairs upon rinsing, preventing the compositions from imparting the hair with sufficient smoothness.

EP 0 615 741 reveals a composition (Example 4), which contains 1.0 wt.% of behenyl trimethyl ammonium chloride and 1.5 wt.% of behenyl alcohol, and which is emulsified in a first step at a temperature of 70°C, and in a second step a high-pressure emulsification at 75°C by use of a high-pressure homogenizer is carried out.

US 4892728 discloses an aliphatic alcohol dispersant (Example 15), which contains 0.1 wt.% of behenyl trimethyl ammonium chloride and 20 wt.% of behenyl alcohol.

US 5951993 discloses a composition (Example 21), which contains 1.5 - 2.0 wt.% of behenyl alcohol and 0.44 - 0.59 wt.% of behenyl trimethyl ammonium chloride being prepared at a temperature of 65-70°C. This temperature is lower than the melting point of behenyl alcohol (72°C).

In US 5213792, a composition (Example 12) is disclosed which contains 0.65 wt.% of stearyl trimethyl ammonium chloride and 1.98 wt.% of stearyl alcohol.

EP 0 407 040 refers to a composition (Example 1), which is characterized by containing 1.0 wt.% of cetyl trimethyl ammonium chloride and 1.0 or 2.0 wt.% of cetyl alcohol. The emulsification is conducted at 80°C, whereas the emulsified product has a phase transition temperature of 66.9°C.

Finally, US 5693317 discloses compositions (Examples 5 and 6), which contain lauryl trimethyl ammonium chloride and lauryl alcohol.

### Disclosure of the Invention

An object of the present invention is to provide a hair cosmetic composition which has excellent stability and is capable of imparting the hair with oil feel and flexibility, particularly, with smoothness during the period from its application until washing-off (rinsing) even if a silicone derivative is added in a large amount.

The present inventors have found that a hair cosmetic composition which is capable of imparting the hair with smoothness during the period from its application to the hair till washing-off (rinsing) and also imparting the hair with moisturized feeling and soft finish and has excellent stability is available by the combined use of a cationic surfactant and a high alcohol having a specific relation to each other.

In one aspect of the present invention, there is thus provided a hair cosmetic composition comprising the following components (A) and (B):
(A) 0.3-5 wt.% of a quaternary ammonium salt represented by the following formula (1): 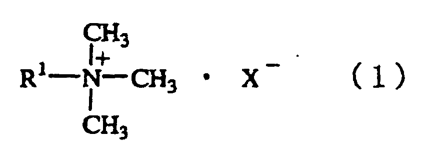
(B) 1-10 wt.% of a higher alcohol represented by the following formula (2):

   R²-OH (2)
[wherein, R¹ and R² each independently represents a C₁₂₋₂₈ aliphatic hydrocarbon group and X⁻ means an anion, with the proviso that the components (A) and (B) may have a distribution in the number of carbon atoms of R¹ and R², respectively; R¹ of the compound showing the maximum content in the component (A) and R² of the compound showing the maximum content in the component (B) are both aliphatic hydrocarbon groups having 22 carbon atoms; and the respective contents of the maximum-content-showing compounds in the components (A) and (B) fall within a range of 70 to 100 wt.%];
wherein the hair cosmetic composition is obtainable by carrying out emulsification at a temperature lower than the phase transition temperature of the hair cosmetic composition to be prepared but not lower than the melting point of the component (B).

In another aspect of the present invention, there is also provided a process for preparing the above-described hair cosmetic composition, which comprises conducting emulsification at a temperature lower than the phase transition temperature of the hair cosmetic composition to be prepared but not lower than the melting point of the component (B).

### Best Mode for Carrying out the Invention

In the formula (1) representing the component (A), R¹ of the quaternary ammonium salt has 12 to 28, preferably 16 to 24, especially 22 carbon atoms. Linear or branched alkyl groups or alkenyl groups, particularly linear alkyl groups are preferred. The distribution of the carbon atoms of R¹ is preferably narrow. The content of the major compound in the component (A) should range from 70 to 100 wt.% (which will hereinafter be described "%", simply), preferably 80 to 100%. Examples of the anion X⁻ include halide ions such as chloride ions and bromide ions, and organic anions such as methosulfate ions, ethosulfate ions, methophosphate ions, ethophosphate ions and methocarbonate ions. Among them, halide ions, particularly, chloride ions are preferred.

Preferred specific examples of the component (A) include cetyltrimethylammonium chloride, stearyltrimethylammonium chloride, arachyltrimethylammonium chloride and behenyltrimethylammonium chloride. From the viewpoints of slick feel upon application and smoothness upon rinsing, behenyltrimethylammonium chloride is preferred.

As the component (A), two or more of the above-described ones may be used in combination. Upon use in combination, the component (A) as a whole should satisfy the above-described condition. The component (A) is incorporated in the hair cosmetic composition of the present invention in an amount of 0.3 to 5%.

In the formula (2) representing the component (B), R² of the higher alcohol has 12 to 28 carbon atoms, preferably 16 to 24 carbon atoms, especially 22 carbon atoms. Linear or branched alkyl groups or alkenyl groups, especially linear alkyl groups are preferred. The distribution of the carbon atoms of R² is preferably narrow. Described specifically, the content of the major compound in the component (B) should fall within a range of 70 to 100%, preferably 80 to 100%.

Preferred specific examples of the component (B) include cetyl alcohol, stearyl alcohol, arachyl alcohol, behenyl alcohol, 2-octyllauryl alcohol, 2-hexyldecyl alcohol, isostearyl alcohol and carnaubyl alcohol (tetracosanol), with behenyl alcohol being especially preferred.

As the component (B), two or more of the above-described ones may be used in combination. Upon use in combination, the component (B) as a whole should satisfy the above-described condition. The component (B) is incorporated in the hair cosmetic composition of the present invention in an amount of 1 to 10%.

In the present invention, R¹ and R² of the compounds exhibiting the maximum contents in the component (A) and (B), respectively are both aliphatic hydrocarbon groups having 22 carbon atoms.

As R¹ and R², a linear alkyl group is preferred, because it brings about excellent effects in slick feel and flexibility during the period from application to the hair to washing-off (rinsing). The linear alkyl group has 22 carbon atoms.

It is preferred to add, to the hair cosmetic composition of the present invention, a quaternary ammonium salt represented by the following formula (3): 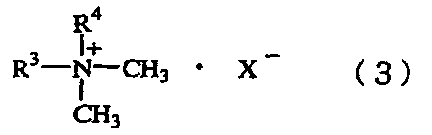 [wherein, R³ and R⁴ each independently represents a hydrogen atom, a C₁₋₂₈ alkyl group or a benzyl group with the proviso that they do not represent a hydrogen atom at the same time, and X⁻ represents an anion similar to that in the formula (1)], because glide and flexibility upon rinsing, smoothness after drying and handling use are improved.

In the formula (3), as the alkyl group of R³ or R⁴, C₈₋₂₄ alkyl groups are preferred.

As the component (C), two or more compounds may be used in combination. The component (C) is preferably added to the hair cosmetic composition of the present invention in an amount of 0.01 to 5%, especially 0.05 to 1%.

Preparation of the hair cosmetic composition of the present invention by emulsification at a temperature lower than its phase transition temperature but not lower than the melting point of the component (B) is performed because it brings about excellent effects in glide or flexibility during the period from its application to the hair until rinsing and a further improvement in stability. Upon emulsification, the phase transition temperature of a composition obtained by mixing only the components (A) and (B) in amounts to be incorporated in the hair cosmetic composition and then, emulsifying the mixture (the mixture of the compound(s) to be added in the largest amount when two or more of the compounds are used as the component (A) or(and) (B)) can be used as an approximate value of the above-described phase transition temperature. When two or more higher alcohols are used as the component (B), the melting point of the major one is used as an approximate value of the above-described melting point of the component (B). Emulsification is effected by propeller stirring, homomixer stirring or static mixer mixing. For emulsification, mixing under stirring is preferably continued until crystals of the component (B) or such substance poor in dispersibility completely dissolves in the emulsion.

The hair cosmetic composition of the present invention is preferred to have a pH (at 25°C) of 2 to 10, especially 3 to 8 from the viewpoint of stability. For pH adjustment, an organic acid such as citric acid or lactic acid, or an inorganic acid may be used.

The hair cosmetic composition of the present invention is prepared in the form of an emulsion.

The hair cosmetic composition of the present invention can be used as a hair rinse, hair conditioner, hair treatment, hair pack, hair cream, conditioning mousse, hair mousse, hair spray, shampoo or leave-on treatment. Especially, use as a hair cosmetic composition such as hair rinse, hair conditioner or hair treatment which is washed off after use is suited.

### Examples

### Example 1

A hair cosmetic composition (hair conditioner) as shown in Table 1 was prepared.

### <Preparation process>

(1) Predetermined amounts of components other than purified water and methylparaben were charged in a 500 mL beaker to give a final preparation amount of 400 g and stirred at 250 rpm by a three-blade propeller under heating.
(2) In heated purified water, methylparaben was dissolved. The mixture obtained in (1) was then added to the resulting solution, followed by emulsification.
   The emulsification temperature was set at a temperature lower than the gel phase transition temperature of a composition which had been obtained by emulsifying a mixture of predetermined amounts (shown in Table 1) of only the components (A) and (B) (when two or more compounds were incorporated as the component (A) or (B), a composition obtained by emulsifying the compound, which had been incorporated in the largest amount, in the corresponding amount) but not lower than the melting point of the component (B) (when two or more compounds were used as the component (B), that incorporated in the largest amount). With regards to the comparative product 3, the emulsifying temperature was determined based on the phase transition temperature of an aqueous solution containing all the compounds added as the components (A) and (B), and the melting point of all the compounds added as the component (B). The gel phase transition temperature and melting point as used herein were measured by a differential scanning calorimeter ("SSC 5200 Series, DSC120", trade name; product of Seiko Instruments, heating rate: 1 °C/min).
(3) The resulting emulsion was cooled to 45°C. A perfume was then added, followed by cooling to 40°C under stirring.

### <Organoleptic Evaluation Test>

A panel of 10 experts conducted organoleptic evaluation while treating a hair bundle (20 g of the female hair subjected to permanent waving treatment once and cut into a length of 30 cm) by the following method.

After shampooing the hair bundle by a commercially available cleanse shampoo, they applied 2 g of a conditioner to the hair bundle and carried out "evaluation upon application". While rinsing the hair bundle under running water of 40°C at 4 L/min, they carried out "evaluation upon rinsing". After drying the hair with a towel and then by a drier, they carried out "evaluation after drying".

### (Evaluation standards)

Based on the following ranking standards: 2 points for good, 1 point for a little good, 0 point for average, -1 point for a little inferior and -2 points for inferior, evaluation was conducted on each item and the results are indicated by an average of the scores of 10 experts.
A: 1.5 or more on average
B: not less than 1.0 but less than 1.5 on average
C: not less than 0.0 but less than 1.0 on average
D: less than 0.0 on average

### <Stability Test>

In a 100 mL glass bottle, 50 g of a hair cosmetic composition was charged, followed by hermetic sealing. The glass bottle was allowed to stand for 30 days in a thermostat set at each of -5°C, 5°C, 40°C and 50°C, in a thermostat subjected to cyclic temperature change once a day within -15 to 60°C, or at room temperature, whereby stability test was conducted.

The hair cosmetic composition which underwent no change in any of the above-described tests was evaluated as A, while the composition which caused decomposition or gelation in at least some of the tests was evaluated as B.

**Table 1**

| (%) | | | | | | | | | | | |
|---|---|---|---|---|---|---|---|---|---|---|---|
| | | Invention product | | | | | Comparative product | | | | |
| | | 1 | 2 | 3 | 4 | 5 | 1 | 2 | 3 | 4 | 5 |
| Component A | Behenyltrimethylammonium chloride | 1.5 | 1.2 | 0.2 | 0.1 | 2.1 | - | 1.2 | 0.75 | - | - |
| | Arachyl trimethylammonium chloride | - | - | - | - | 0.3 | - | - | - | - | - |
| | Stearyltrimethylammonium chloride | - | 0.2 | 1.2 | 0.1 | 0.1 | 1.5 | 0.2 | 0.75 | 2.0 | - |
| | Cetyltrimethylammonium chloride | - | 0.1 | 0.1 | 1.3 | - | - | 0.1 | - | - | 1.0 |
| Component B | Carnaubyl alcohol | - | - | - | - | 0.1 | - | - | - | - | |
| | Behenyl alcohol | 5.0 | 4.25 | 0.5 | 0.2 | 8.7 | 5.0 | - | 2.5 | - | - |
| | Arachyl alcohol | - | - | - | - | 1.0 | - | - | - | - | - |
| | Stearyl alcohol | - | 0.6 | 4 | 0.1 | 0.2 | - | 3.5 | 2.5 | 1.5 | 4.0 |
| | Cetyl alcohol | - | 0.15 | 0.5 | 4.5 | - | - | 1.5 | - | 1.5 | - |
| Other components | Propylene glycol | 2.0 | 2.0 | 3.0 | 2.0 | 2.0 | 2.0 | 2.0 | 3.0 | 2.0 | 2.0 |
| | Isopropyl palmitate | 1.0 | 1.0 | 2.0 | 1.0 | 1.0 | 1.0 | 1.0 | 2.0 | - | - |
| | Methyl polysiloxane (150000 mPa·S) | 2.0 | 1.0 | 2.0 | 2.0 | 2.0 | 2.0 | 1.0 | 2.0 | - | - |
| | Methylparaben | 0.1 | 0.1 | 0.1 | 0.1 | 0.1 | 0.1 | 0.1 | 0.1 | 0.1 | 0.1 |
| | Perfume | 0.3 | 0.3 | 0.3 | 0.3 | 0.3 | 0.3 | 0.3 | 0.3 | 0.3 | 0.3 |
| | Purified water | Balance | Balance | Balance | Balance | Balance | Balance | Balance | Balance | Balance | Balance |
| Phase transition temperature (°C) | | 85 | 84 | 75 | 67 | 84 | 81 | 76 | 79 | 74 | 72 |
| Heating temperature for emulsification upon preparation (°C) | | 75 | 75 | 65 | 60 | 75 | 75 | 65 | 75 | 65 | 65 |
| Effects | Smoothness upon application | A | A | B | B | A | D | D | C | D | D |
| | Smoothness upon rinsing | A | A | B | B | A | D | C | C | D | D |
| | Softness after drying | A | A | A | A | A | D | C | C | C | C |
| | Moisturized feeling after drying | A | A | B | B | A | D | C | C | D | D |
| Stability | | A | A | A | A | A | B | A | A | A | A |

As the components in Table 1, the below-described ones were employed, which will be applied equally to the subsequent examples.

| Component (maker) | Alkyl purity | Melting point |
|---|---|---|
| Behenyltrimethylammonium chloride (Kao) | >98% | - |
| Arachyltrimethylammonium chloride (Kao) | >95% | - |
| Stearyltrimethylammonium chloride (Tokyo Kasei Kogyo) | >97% | - |
| Cetyltrimethylammonium chloride (Tokyo Kasei Kogyo) | >98% | - |
| Carnaubyl alcohol (Tokyo Kasei Kogyo) | >98% | 74°C |
| Behenyl alcohol (Tokyo Kasei Kogyo) | >95% | 72°C |
| Arachyl alcohol (Tokyo Kasei Kogyo) | >96% | 65°C |
| Stearyl alcohol (Tokyo Kasei Kogyo) | >99% | 59°C |
| Cetyl alcohol (Tokyo Kasei Kogyo) | >98% | 50°C |

It has been found that the hair cosmetic compositions (hair conditioners) of the present invention were excellent in smoothness upon application and rinsing, and softness and moisturized feeling after drying and were stable.

### Example 2 Hair rinse

| | (%) |
|---|---|
| Behenyltrimethylammonium chloride | 1.3 |
| Stearyltrimethylammonium chloride | 0.2 |
| Behenyl alcohol | 4.5 |
| Cetyl alcohol | 0.5 |
| Propylene glycol | 3.0 |
| High polymerization polyethylene glycol (n=4500) | 0.1 |
| Isopropyl myristate | 0.5 |
| Hydroxyethyl cellulose | 0.1 |
| Isostearyl glyceryl ether | 0.2 |
| Camellia oil | 0.3 |
| Aqueous solution (50%) of malic acid | 0.5 |
| Methyl polysiloxane (5000 mPa·s) | 1.0 |
| Methylparaben | 0.1 |
| Perfume | 0.3 |
| Purified water | Balance |
| Total | 100.0 |

### Example 3 Hair conditioner

| | (%) |
|---|---|
| Behenyltrimethylammonium chloride | 2.0 |
| Stearyltrimethylammonium chloride | 0.5 |
| Dialkyldimethylammonium chloride * | 0.5 |
| Behenyl alcohol | 6.5 |
| Cetyl alcohol | 1.5 |
| Propylene glycol | 1.0 |
| Ethyl carbitol | 1.0 |
| Benzyloxyethanol | 0.3 |
| Dipentaerythritol fatty acid ester (hydroxystearic acid · stearic acid · rhodinic acid) | 0.2 |
| Octyldodecyl myristate | 1.0 |
| Hydroxyethyl cellulose | 0.2 |
| Olive oil | 0.5 |
| Amodimethicone emulsion ("SM8702C", trade name; product of Dow Corning Toray) | 0.3 |
| Dimethyl silicone emulsion ("BY22-019", trade name; product of Dow Corning Toray) | 1.5 |
| Aloe extract | 0.05 |
| Methylparaben | 0.1 |
| Perfume | 0.3 |
| Purified water | Balance |
| Total | 100.0 |

| | |
|---|---|
| *: a dialkyl quaternary ammonium salt derived from oxo alcohol (branching ratio: 20%) having 12 to 15 carbon atoms (effective ingredient: 55%) | |

### Example 4 Hair conditioner

| | (%) |
|---|---|
| Behenyltrimethylammonium chloride | 3.36 |
| Stearyltrimethylammonium chloride | 0.5 |
| Behenyl alcohol | 10.0 |
| 1,3-Butylene glycol | 2.0 |
| Ethyl carbitol | 0.5 |
| Benzyl alcohol | 0.3 |
| Vaseline | 0.5 |
| Glyceryl trioctanoate | 1.0 |
| Hydroxyethyl cellulose | 0.2 |
| Meadow foam oil | 0.5 |
| Methyl polysiloxane (150000 mPa·s) | 2.0 |
| Poly(oxyethylene·oxypropylene)methyl polysiloxane copolymer ("KF352A", trade name; product of Shin-etsu Chemical) | 0.5 |
| Casein | 0.1 |
| Methylparaben | 0.1 |
| Perfume | 0.2 |
| Purified water | Balance |
| Total | 100.0 |

It has been found that the hair cosmetic compositions obtained in Examples 2 to 4 imparted the hair with oil feel and flexibility and also with smooth touch during the period from application to the hair until the completion of rinsing.

## Claims

1. A hair cosmetic composition comprising the following components (A) and (B):
(A) 0.3 - 5 wt.% of a quaternary ammonium salt represented by the following formula (1): 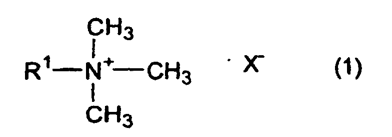
(B) 1 - 10 wt.% of a higher alcohol represented by the following formula (2):
R²-OH (2)
wherein R¹ and R² each independently represents a C₁₂₋₂₈ aliphatic hydrocarbon group and X⁻ means an anion, with the proviso that the components (A) and (B) may have a distribution in the number of carbon atoms of R¹ and R², respectively; R¹ of the compound showing the maximum content in the component (A) and R² of the compound showing the maximum content in the component (B) are both aliphatic hydrocarbon groups having 22 carbon atoms; and the respective contents of the maximum content showing compounds in the components (A) and (B) fall within a range of 70 to 100 wt.%, wherein the hair cosmetic composition is obtainable by carrying out emulsification at a temperature lower than the phase transition temperature of the hair cosmetic composition to be prepared but not lower than the melting point of the component (B).

2. A process for preparing a hair cosmetic composition as claimed in Claim 1, which comprises carrying out emulsification at a temperature lower than the phase transition temperature of the hair cosmetic composition to be prepared but not lower than the melting point of the component (B).

## Patentansprüche

1. Haarkosmetische Zusammensetzung, umfassend die folgenden Komponenten (A) und (B):
(A) 0,3 - 5 Gew.-% eines quaternären Ammoniumsalzes mit der folgenden Formel (1) : 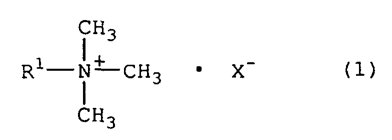
(B) 1 - 10 Gew.-% eines höheren Alkohols mit der folgenden Formel (2):
R²-OH (2)
worin R¹ und R² jeweils unabhängig eine aliphatische C₁₂₋₂₈ Kohlenwasserstoffgruppe sind und X- ein Anion ist, mit dem Vorbehalt, dass die Komponenten (A) und (B) eine Verteilung bezüglich der Anzahl der Kohlenstoffatome von R¹ bzw. R² haben können; R¹ der Verbindung, die den maximalen Gehalt in der Komponente (A) zeigt, und R² der Verbindung, die den maximalen Gehalt in der Komponente (B) zeigt, jeweils aliphatische Kohlenwasserstoffgruppen mit 22 Kohlenstoffatomen sind; und die jeweiligen Gehalte des maximalen Gehaltes, die die Verbindungen in den Komponenten (A) und (B) zeigen, innerhalb eines Bereichs von 70 bis 100 Gew.-% fallen, worin die haarkosmetische Zusammensetzung erhältlich ist durch Durchführen einer Emulgierung bei einer Temperatur, die niedriger ist als die Phasenübergangstemperatur der herzustellenden haarkosmetischen Zusammensetzung, aber nicht niedriger als der Schmelzpunkt der Komponente (B).

2. Verfahren zur Erzeugung einer haarkosmetischen Zusammensetzung wie in Anspruch 1 definiert, umfassend das Durchführen einer Emulgierung bei einer Temperatur, die niedriger ist als die Phasenübergangstemperatur der herzustellenden haarkosmetischen Zusammensetzung, aber nicht niedriger ist als der Schmelzpunkt der Komponente (B).

## Revendications

1. Composition cosmétique pour les cheveux comprenant les composants (A) et (B) suivants :
(A) de 0,3 à 5 % en poids d'un sel d'ammonium quaternaire représenté par la formule (1) suivante : 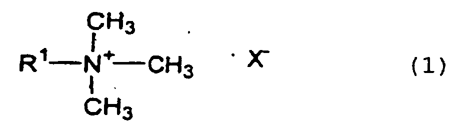
(B) de 1 à 10 % en poids d'un alcool supérieur représenté par la formule (2) suivante :
R²-OH (2)
dans lesquelles R¹ et R² représentent chacun indépendamment un groupe hydrocarbure aliphatique en C₁₂₋₂₈ et X⁻ signifie un anion, à la condition que les composants (A) et (B) puissent avoir une distribution dans le nombre d'atomes de carbone de R¹ et R², respectivement ; R¹ du composé montrant la teneur maximale dans le composant (A) et R² du composé montrant la teneur maximale dans le composant (B) sont les deux des groupes hydrocarbures aliphatiques ayant 22 atomes de carbone ; et les teneurs respectives des composés montrant une teneur maximale dans les composants (A) et (B) tombent dans une gamme de 70 à 100 % en poids, où la composition cosmétique pour les cheveux est susceptible d'être obtenue en effectuant une émulsification à une température inférieure à la température de transition de phase de la composition cosmétique pour les cheveux qui est préparée mais pas inférieure au point de fusion du composant (B).

2. Procédé pour préparer une composition cosmétique pour les cheveux comme revendiquée dans la revendication 1, qui comprend d'effectuer une émulsification à une température inférieure à la température de transition de phase de la composition cosmétique pour les cheveux qui est préparée mais pas inférieure au point de fusion du composant (B).
